# EUROPEAN PATENT APPLICATION

(11) **EP 2 574 349 A1**
(43) Date of publication of application: **03.04.2013**
(21) Application number: 11183232.5
(22) Date of filing: 29.09.2011
(51) Int. Cl.: A61L 26/00, A61L 27/22

(54) **Therapeutic use of gelatin hydrogels with a gel-sol transition at body temperature**

(71) Applicant: Biorigen S.r.l, 16149 Genova (IT); Lorandi, Christian, 38060 Aldeno (TN) (IT); Motta, M Antonella, 38050 Tenna (TN) (IT); Migliaresi, Claudio, 38050 Tenna (IT)
(72) Inventor: Lorandi, Christian, 38060 Aldeno (TN) (IT); Motta, Antonella, 38050 Tenna (TN) (IT); Migliaresi, Claudio, 38050 Tenna (TN) (IT); Cancedda, Ranieri, 16145 Genova (IT); Mastrogiacomo, Maddalena, 16132 Genova (IT); Muraglia, Anita, 17024 Finale Ligure (GE) (IT)
(74) Representative: Capasso, Olga

(57) **Abstract**

The invention regards the use of gelatin hydrogels that are solid at room temperature and melt or present a gel-sol transition at temperatures near the body temperature, so assuming a liquid state when put contact with body, implanted in the body or heated at temperatures compatible with the body.

The gelatin-based hydrogels may contain biologically active agents and/or cells that after hydrogel melting or gel-sol transition, are fast released in situ, thus explicating the therapeutic and biological action.

The gelatin-based hydrogels, containing or not containing biologically active agents, may be solid at the temperature of the skin (33°C) and melt when water or aqueous solution at a temperature compatible to the body is poured onto for an easy removal of the hydrogels.

## Description

### FIELD OF INVENTION

The invention relates to gelatin hydrogels that are solid at room temperature and melt at temperatures near the mammalian body temperature and uses thereof. The hydrogels assume a liquid state when placed in contact with the body, when implanted into the body or when heated at temperatures compatible with the mammalian body temperature. The gelatin hydrogel may be in the form of a membrane, film, fiber, woven and non-woven fabric, foam, microbead and particle. The gelatin hydrogels may also contain biologically active agents and/or cells and/or stem cells and/or conditioned cells which are encapsulated and/or entrapped and/or loaded therein. Upon gel melting, such additional components are rapidly released in situ, thus exerting their therapeutic and biological action.

### BACKGROUND OF INVENTION

Collagens have a tertiary unique structure given by amino acid sequences. The collagen molecules of different sources consists of three polypeptide chains twined around one another as in a three-stranded rope.

Collagen represents the primary structural protein accounting for approximately 30% of all vertebrate body protein. More than 90% of the extracellular protein in the tendon and bone and more than 50% in the skin consist of collagen. Although most of the scaffolding in mammals is composed of collagen, the collagenous spectrum ranges from Achilles tendons to the cornea. Hence, different collagen types are necessary to confer distinct biological features to the various types of connective tissues in the body. Currently at least 13 types have been isolated which vary in the length of the helix and the nature and size of the non-helical portions.

In the manufacture of gelatin, treatment of the animal collagen raw material with dilute acid or alkali results in partial cleavage of these cross-links; the structure is broken down to such an extent that "warm water-soluble collagen", i.e. gelatin, is formed. This chemical hydrolysis can be supplemented or even replaced by the use of enzymes.

The goal of the gelatin manufacturer is to carry out a controlled partial hydrolysis of the cross-links and peptide bonds of the original collagen structure and to obtain the ideal molecular weight distribution of gelatin for the application envisaged. The viscosity of a gelatin solution, for example, correlates relatively well with the proportion of high molecular weight components.

Gelatins are widely used as biomaterials in drug delivery, pharmaceutical applications and regenerative medicine. Moreover, gelatin is biodegradable, bioreabsorbable, no-toxic, and exhibits weak immunogenicity and superior biocompatibility compared to synthetic polymers and with other natural polymers.

The attractiveness of gelatin as a biomaterial rests largely on the view that it is a natural material of low immunogenicity and is therefore seen by the body as a normal constituent rather than foreign matter. Gelatin can be processed into a number of forms such as films, membranes, sheets, tubes, capsules, beads, nets, sponges, powders, fleeces, injectable solutions and dispersions, micro and nano-spheres, single unit devices, or other geometrical forms, all of which have found use in medical practice. Furthermore, gelatin has been applied for drug delivery in a variety of applications, such as ophthalmology, wound and burn dressing, tissue repair and tissue engineering, inserts and physical barrier shields.

Cross-linked gelatins have also been used to confer mechanical firmness and collagenase resistance by introduction of exogenous cross-linking into the molecular structure. In fact, to address some of the drawbacks in gelatin based applications due to the poor mechanical strength of gelatin, improvement of their physical, chemical and biological properties has often been needed. However, a major handicap of chemical cross-linking agents is the potential toxic effect of residual molecules and/or compounds formed during in vivo degradation.

Alternative physical methods have been pursued, including dry heat, exposure to ultraviolet or gamma-irradiation. In these cases, the drawback is that gelatin becomes partially denatured by these physical treatments.

Extensive reviews on collagens and gelatin, their sources, physical and chemical properties, treatments and applications are in Friess W., Eur. J. Pharm. Biopharm. 45, 113-136, 1998, Lee C.H. et al., Int. J. Pharm. 221, 1-22, 2001, and Nair et al., JITPS 1(7), 288-304, 2010.

Bioactive molecules may be loaded by different ways into the gelatin matrix which can be both in a solid state or in an hydrogel form. The hydrogel is typically obtained by solidification from a solution or by co-precipitation in suitable conditions. The bioactive molecules are then entrapped in the interstices of the gelatin matrix, which acts as a reservoir.

The release of the bioactive molecules occurs from gelatin hydrogels, prepared with different methods, size and form and then implanted in the human body site. The gelatin hydrogels can also be generated *in situ* from a solid matrix when placed in contact with an aqueous environment by a swelling mechanism. In both cases, the final aim is to provide a hydrogel, single unit or multi-particulate, from which a bioactive molecule can be released. The release may occur by simple diffusion in the case of cross-linked hydrogels or by a more complex mechanism of diffusion including concurrent erosion by and dissolution in aqueous solutions of the outer layer of the collagen matrix.

Many delivery systems of bioactive molecule have been developed by exploiting the properties of single unit and multi-particulate hydrogels.

Gelatin film, or sheet, or disc has been used for the treatment of tissue infection, such as infected corneal tissue or liver cancer, and wound healing by placing in contact the hydrogel with the part to be treated.

Gelatin sponges have been very useful in the treatment of severe burns and as a dressing for many types of wounds, such as pressure sores, donor sites, leg ulcers and decubitus ulcers as well as for in vitro test systems.

Gelatin gel micro-particles have primarily been used for injectable systems. Gelatin micro-particles containing the bioactive molecules are injected into the tissue site of interest, the molecule then being released in a controlled manner.

Minipellets made of gelatin, typically in rods, have also been developed.

The rod (minipellet) is small enough to be injected into the subcutaneous space through a syringe needle.

Physically resistant gelatin gels, also containing bioactive agents, have been applied more recently as bone substitutes and, more in general, bioengineered tissues.

In all these applications, the gelatin gels must be physically and mechanically sufficiently resistant to stay in situ for a prolonged time period. They must also control the release of the bioactive agents. Consequently, the gel-sol transition temperature of the gelatin hydrogel, also called as the melting temperature of the gel, must be higher than the body temperature, namely at least 43-45°C.

EP 0518697 discloses single and multiple layer gelatin films to improve the sustained release of pharmaceuticals, specifically of growth factors. For multilayer preparation, the films are attached together by evenly applying pressure. The document describes that platelet derived growth factor was released at constant rate for up to 100 hours and improved wound healing in vivo.

US 4,865,846 discloses an eye treatment system which includes particles of bio-erodible material (i.e. gelatin), which are suspended in a liquid carrier or ointment carrier having at least one drug therein and having a pH acceptable to the eye. The document describes that an administration containing from about 3 to 6 particles of drug loaded cross-linked collagen has provide continuous delivery of tobramycin for about 6 hours, which is a very long time for an ophthalmic delivery.

EP 0069260 describes gelatin insert containing active ingredient for introduction in bone or soft parts, where collagen has a factor of nitrogen to hydroxylproline lower than 3-5 and is made in form of sheet and optionally wound into a rod. The document reports that an insert containing 100 mg gentamycin has been placed in shinbone, where the drug has been released up to the re-absorption of the insert up to three weeks.

US 4,659,572 discloses a burn wound-adherent dressing material composed of a complex with gelatin and a water-soluble resin aimed at controlling water-loss and minimizing the ingress of exogenous micro-organisms. The gel protection remains on the wound 7 or more days and is then removed after starting of healing process, when skin grafting is allowed.

In US Patent Application 2005/0036955, a moldable, bioresorbable, biocompatible, non-allergenic cross-linked gelatin derivative dressing for the prevention of post extraction alveolar osteitis pain is disclosed along with methods for use of the gel. The dressing is placed at the time of surgery acting as a bone covering obtundant and physiologic scaffolding for the conduction of normal alveolar bone healing sequence of fibroblast ingrowth, blood vessel formation, and reossification of the extraction site defect.

US 5,895,412 discloses an apparatus and method for effecting and enhancing wound closure in tissue by flowing a heated sealant flow, such as gelatin, over the wound. This creates an effective barrier against further blood leakage and, upon cooling, the flow readily adheres to the tissue to seal the wound.

For wound protection and skin healing, extensive investigation has been carried out on the hydrophilic and water absorbing properties of gelatin as a clinical wound dressing (Takahashi H. et al., Tokushima. J. Exp Med.40(3-4), 159-67 and 169-75, 1993).

The use of gelatin gels and cross-linked collagen gelatins has been investigated, so far, as "depot" systems which slowly release the biologically active agents for a prolonged release. However, degradation of the gelatin matrix in such gels is observed. The degradation occurs after a long period of time and in any form such as enzymatic, erosion, dissolution. The degradation depends on the site of application (i.e. from 6-8 hours in ophthalmic applications, where the elimination is in general very fast, to 7-10 days in other body districts, such as skin).

The common characteristics of gelatin-based systems are that they are solid at 37 °C and have a melting temperature, i.e. a gel-sol transition temperature, much higher than the body temperature, at least 43-45°C or more. The commercial advertisements for gelatin addressed also to this point. They report that the melting point of gelatin is to be considered as a quality index. In other words, a higher melting point is advertised as both an index of good gelatin and a sign of good stability. It is also advertised that gelatins will lose activity, denature and die when the temperature is substantially higher than their melting point. Thus, the lost of activity will mainly occur with low melting gelatins. Therefore, the right choice for the gelatin to be used in the above medical applications is to select the one with a melting point as high as possible.

There is still the need for an improved gelatin hydrogel which allows:
a. the release of a high amount of biologically active agents in a short period of time (i.e. immediate or almost immediate release); and/or
b. the immediate contact of the biologically active agents with the surface of the organ to be treated, so that the agent can immediately exert its therapeutic and biological function; and/or
c. the easy removal of the gelatin gel films, or other forms, from wounded skin (burning, bedsore) without any damage or pain.

Traditional or prior art gels, that are solid at 37 °c, act as a matrix for controlled release but do not act for an almost immediate release.

In topical uses, such as in wound healing, it is better to have the release of a high concentration of the adjuvant agents (such as PRP) in a short time. Further, if the gel melts naturally at body temperature or by pouring or washing with a warm solution (38-40°C), the wound is not affected. By contrast, if the gel is solid on the wound, when it is substituted, it adheres to the wound and its removal provokes both pain and the reopening of the wound.

For instance there is the need for a gelatin hydrogel allowing the contact between platelet derived components, including, but not limited to platelet rich plasma (PRP) and platelet lysate (PL), platelet poor plasma (PPP), cryoprecipitate (CRYO) alone or together with other therapeutic agents and wounded skin or organ lesion, wherein the platelet components can release growth factors and exert their healing properties. In particular, in skin treatment, an immediate contact of all available platelet components with the skin lesions would help repairing them and would favor the growth of regenerated skin. If the skin lesion is continuously in contact with fresh platelet components (including PRP and PL) by mean of a suitable delivery system, rapid regeneration will occur, even when replacing the delivery system.

There is also the need for a delivery system or gelatin hydrogels in which cells and/or stem cells and/or cell conditioned culture medium are encapsulated and/or entrapped and/or loaded into.

There is also the need for a delivery system or gelatin hydrogels, either single unit or multi-particulate, that can be surgically inserted or injected, as a suspension, in the organ to be treated where rapidly release the active principle. Such system would be typically used in anti-cancer and/or anti-microbial and/or antibiotic and/or anti-inflammatory therapy and/or in regenerative medicine and advanced therapies (i.e. cells and/or conditioned culture medium).

For instance, in cancer therapy, depot systems and, sometimes, targeted therapeutics allow the controlled release of the therapeutic drugs preferably in the site of action. The well-known cytotoxic systemic effects are then avoided. However, the total amount of drug released might be insufficient to properly tackle the tumor mass. Therefore, there is the need for a delivery system which releases the drug only in the target site in order to reduce to a minimum free systemic circulation, but also able to provide enough drug so it can exert its local action.

There is also the need for a gel film that can be easily placed as a protecting barrier against infection on skin wounds or lesions that is not painful to remove.

The gel may contain biologically active agents and may be used on skin wounds or lesions due to burning or to relieve bedsore or metabolic ulcers.

Often, removal of the film is painful given the adhesion of the gel onto the wounds. A hydrogel system which is in a gel state at the temperature of the skin (33°C) but "melts" when water at a temperature compatible to the body (36-38°C) is poured onto it, would be removed easily and without pain when substituted.

Moreover, the hydrogel system need to be composed of biocompatible, bioresorbable and biodegradable materials, preferably from natural sources (not synthetic) and not chemically modified, such as cross-linked.

Among the materials, pure gelatin (not cross-linked) has proven to possess the characteristics of being biocompatible, bioresorbable and biodegradable. It also has a better biocompatibility than other natural products, such as albumin (Lee C.H. et al., Int. J. Pharm. 221, 1-22, 2001).

The gelatin-based systems taught in the state of the art are not suitable for a medical use requiring an immediate or almost immediate release of the biologically active agents. They are also not suitable for a direct contact with the surface of the organ to be treated and for a rapid bioresorbtion of the gelatin matrix. In fact, in the prior art system, the release is sustained for a long period of time, in general days, and the hydrogel matrix, when not cross-linked, undergoes physical degradation by erosion and/or dissolution of the polymer chains.

### DESCRIPTION OF THE INVENTION

The above problems have been solved in the present invention by the use of gelatin hydrogels that are solid at room temperature and melt at temperatures near the mammalian body temperature. Thus, the gel assumes a liquid state when placed into contact with the body, when implanted in the body or when heated at temperatures compatible with the mammalian body temperature. The gelatin gel may be in the form of membranes, films, or associated to fibers, woven and non-woven fabrics, foams, micro-beads and particles. The products melt or present gel-sol transition rates varying from minutes to hours depending on the collagen source, on the composition and on preparation methods, as well.

The gelatin hydrogels may contain biologically active agents and/or cells that are rapidly released in situ after melting, thus exerting their therapeutic and biological action.

The gelatin-based hydrogels of the present invention may contain biologically active agents and, when placed in sites which allow the gel-sol transition, they "melt" immediately thus releasing the agents in situ which in turn exert their therapeutic and biological action.

The gelatin-based hydrogels of the present invention containing or not containing biologically active agents, are solid at the temperature of the skin (33°C) and melt when water or an aqueous solution at a temperature compatible to the body (36-38°C) is poured onto, which provide for an easy removal of the hydrogels.

It is therefore an object of the invention a gelatin gel comprising between 5 and 25 % w/w of gelatin able to melt at a temperature between 34°C and 39°C for medical use. Preferably, the gelatin is dissolved in a solvent comprised in the group of: water, blood and/or plasma derivatives, conditioned cell culture medium, cell suspension containing cell culture medium, therapeutically and/or biologically active agent solution.

Still preferably the blood and/or plasma derivatives is selected in the group of: Platelet-Rich-Plasma (PRP), Platelet-Lysate (PL), and/or Platelet Poor Plasma (PPP), and/or Cryoprecipitate (CRYO).

In a preferred embodiment the conditioned cell culture medium is conditioned by human stem cells.

Preferably the human stem cells are human amniotic liquid derived stem cells.

In a preferred embodiment the gelatin gel as described above is for use as a tissue regenerating agent.

Preferably the tissue is skin, derma and/or connective tissue.

In a preferred embodiment the gelatin gel of the invention is for use as a tissue regenerating agent further to a necrosis or a burning event.

Preferably, the gelatin gel of the invention is for use as a tissue regenerating agent for wound or bed sores healing.

Preferably, the gelatin gel of the invention is for topical administration.

Still preferably, the gelatin gel of the invention is for use as an anti-adhesion agent and/or as a barrier.

The anti-adhesion characteristic derives from the fact that the gel for instance in the form of a film can act as a barrier in respect of two tissues in contact, preventing their healing. Then, the gel liquefies and disappears.

Preferably the therapeutically and/or biologically active agent solution is a growth factor.

Still preferably the therapeutically and/or biologically active agent solution is selected from the group of: an anti-tumor agent, an anti-bacterial and/or an anti-microbial agent, an anti-viral agent, an anti-inflammatory agent.

In a preferred embodiment the gelatin gel of the invention is in a flat shape, a tridimensional shape.

Still preferably the gelatin gel of the invention is dehydrated or lyophilized.

The gelatin-based hydrogels can be as a single unit or multi-particulate. The single unit systems are implanted in situ by surgical operation or minimally invasive technique (i.e. sub-cutaneous), or placed on the wounded skin or inserted in any site of therapeutic interest. The multi-particulates are injected in situ by large needle syringes or delivered by other suitable means.

The gelatin-based hydrogels possess suitable mechanical properties to be easily handled and manipulated.

According to the invention, the gelatin -based hydrogels melt in the range of body temperature in order to exert their action. The melting temperature ranges from 32°C to 42°C, preferably from 35° to 39°C.

According to the invention, the gelatin -based hydrogels have a content of water which depends on the final use, and on the physical resistance to stress needed during manipulation. In fact, the physical resistance of the hydrogels should be higher when they are applied externally, i.e. on the skin surface, and lower when applied in an internal part of the body.

For both the external (skin) and internal use of single unit hydrogels in whatsoever form and size, such as films, sheets and discs, etc., the gelatin/water relative ratio ranges from 5/95 w/w to 50/50 w/w, preferably ranges from 10/90 w/w to 30/70 w/w.

In multi-particulate systems, such as particle, beads, vesicles, etc., the gelatin/water relative ratio ranges from 1/99 w/w to 50/50, preferably ranges from 2/98 w/w to 25/75 w/w.

According to the invention, the source of gelatin can be any organism capable of providing a material with the melting properties required. As a not limitative example, sources are vertebrate animals such as porcine (i.e. pig), bovine (i.e. cow), avian (i.e. chicken), and caprine (i.e. goat), marine fish and invertebrates such as shark, stingray, codfish, salmon, jelly-fish, cattle-fish, squid, octopus.

According to the invention, the gelatin-based single unit hydrogels have different forms and shapes. As a not limitative example, hydrogels are flat, substantially bidimensional forms, such as films, sheets and discs, sponges, porous sponges, or standard tri-dimensional forms, such as tablet, mini-tablet, mini-pellet, beads, nets, or other geometrical forms which are shaped and adapted by the end user (i.e. cut) for the final place of use/implant.

According to the invention, the single unit hydrogels are prepared in different ways. As a not limitative example, the single unit hydrogels are prepared by phase separation from a warm collagen solution (higher than 45-50°C) by mean of temperature cooling or addition of phase-separating agents such as lyotropic salts or addition of water-capturing agents, to get a gelatin hydrogel. Such intermediate gelatin hydrogel is then heat dried or lyophilized to get a dry gelatin matrix that after reconstitution (water absorption) gives rise to the final gelatin hydrogel. The dried forms have also the advantage to be more stable and, consequently, have a longer shelf-life.

According to the invention, the gelatin-based hydrogels are placed on the wound skin (i.e. lesions, burns, superficial inflammation), or surgically implanted in contact with the target organ(s), or implanted sub-cutaneously, or placed in any place (i.e. buccal delivery) where the systems have to explicate their therapeutic action.

According to the invention, the gel-sol transition of the single unit gelatin hydrogels occurs in less than 96 hours, preferably less than 36 hours, most preferably less than 6 hours, as a function of application.

According to the invention, the gelatin multi-particulate systems are, as a not limitative example, micro-spheres, micro-beads, micro-pellets in different forms (i.e. rods), vesicles and nano-composites, and other geometrical forms. They are prepared in different ways. As a not limitative example, the micro-particulates are prepared by adding water, at room temperature or lower, on the gelatin powder, as bulk or previously ground to have a narrower particle size distribution, and stirring the suspension. The micro-particulates can also be prepared by means of micro-fluidic systems through the addition of a separation-inducing oil to a warm collagen gelatin solution and successive removal of the oil.

According to the invention, the gelatin multi-particulate systems are delivered in situ by injection using a large hollowed needle.

According to the invention, the gel-sol transition of the gelatin multi-particulate systems occurs in less than 48 hours, preferably less than 24 hours, most preferably less than 6 hours.

According to the invention, the gelatin-based systems are suitable for many therapies which need an immediate or almost immediate release of the therapeutically and biologically active agents in situ, in contact with the organ where they explicate the action. As a not limitative example, suitable therapies includes the field of skin lesions and skin repair, such as burning and wounds, oncology, anti-inflammatory, anti-bacterial, anti-infectious, and anti-microbial.

The biologically active agents can be included into a gelatin hydrogel also having a temporarily function of physical barrier, i.e. between two or more organs. The gelatin hydrogels would have in this case both the functions of biologically active agent carrier and separation barrier between organs.

According to the invention, the therapeutically and biologically active agents to be loaded in and carried by the gelatin-based systems are, as a not limitative example, blood and plasma derivatives, such as platelet rich plasma, also including co-agents like growth factors; anti-tumor drugs, such as bleomycins, anthraquinone (anthracycline) series carcinostatics such as adriamycin (doxorubicin), daunomycin (daunorubicin), aclarubicin, amrubicin, idarubicin, epirubicin, pirarubicin, and mitoxantrone, mitomycins, actinomycins, camptothecines such as irinotecan, cisplatins, streptozotocin, 5-fluorouracil (5-FU) and derivatives thereof, pirarubicin, dacarbazine and pharmacologically acceptable salts, alkeran, hydrea, avastin, busulfan, cis-platinum, carbo-platinum, methotrexate, cytoxan, xelodan, taxol, paclitaxel, docetaxel, erlotinib, lapatinib, leustatin, genticabine, fludarabine, herceptin, rituxan, ifosfamide, navelbine, topotecan, velban, vincristine; anti-bacterial (antibiotic) and microbial drug, such as oxophosphoric acid, ormetoprim, trimethoprim, sulfonamids, phosphomycin, penicillin-series antimicrobial drugs, cephalosporin-series antimicrobial drugs, vancomycin, tetracycline-series antimicrobial drugs, rifampicin, fluoroquinone-series, furazoldone, gentamycin, lincomycin; anti-infectious drugs, such as antifungal (itraconazole, ketoconazole, myconazole, imidazole, triazole, clotrimazole, butoconazole, ciclopirox), anti-viral (acyclovir, famciclovir, ritonavir, oseltamivir, valacyclovir, amprenavir, lopinavir, nelfinavir, atanazavir, indinavir, saquanavir), local anti-infective (chlorexidine); anti-inflammatory drugs, such as acetylsalicylic acid, ibuprofen, ketoprofen, , naproxen, fenbufen, fenoprofen, flur-biprofen, etodolac, ketorolac, paracetamol (acetaminophen), diclofenac, piroxicam, meloxicam, tenoxicam, nimesulide, COX2-inhibitors (celecoxib, rofecoxib, etoricoxib, valdecoxib), indomethacin, azapropazone, phenylbutazone, nabumetone, diflunisal, tiaprofenic acid, mefenamic, sulindac, tolfenamic acid. The biologically active agents may also be growth factors or other biologically active molecules which exert an action of protection and/or stimulation on wounds and/or the surgically operated organs. The biologically active agents may also be cells and/or stem cells and/or cell conditioned medium which are encapsulated and/or entrapped and/or loaded in the gelatin hydrogels.

The invention is now described by reference to the following non-limiting examples referring to the following figures.

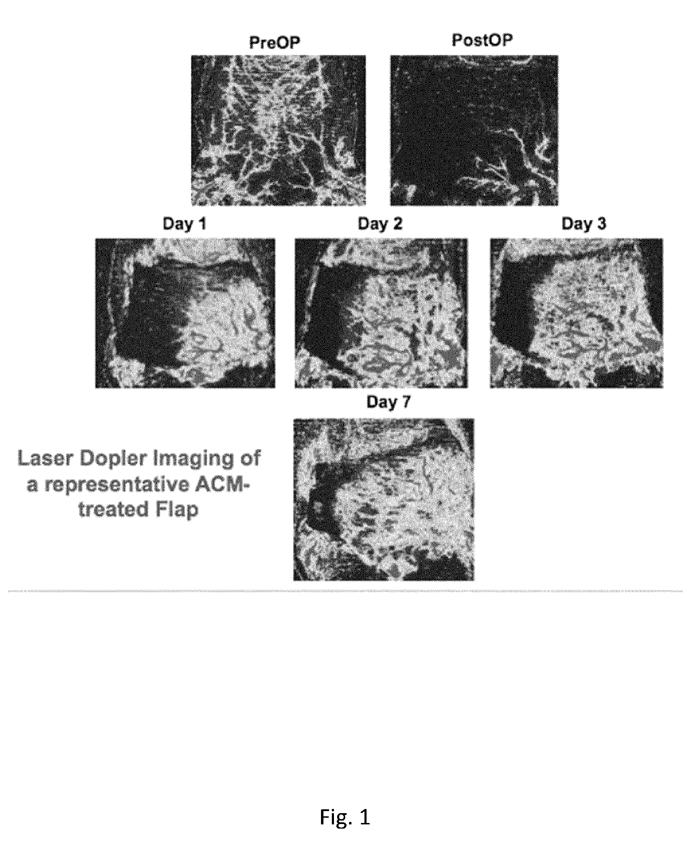

Fig. 1 Laser Dopler Imaging of flaps before operation (PreOP) and at different times after ligation of the epigastric bundle for the conditioned medium containing gelatin membrane (ACM) treated group of animals and in the control group (CTRL)

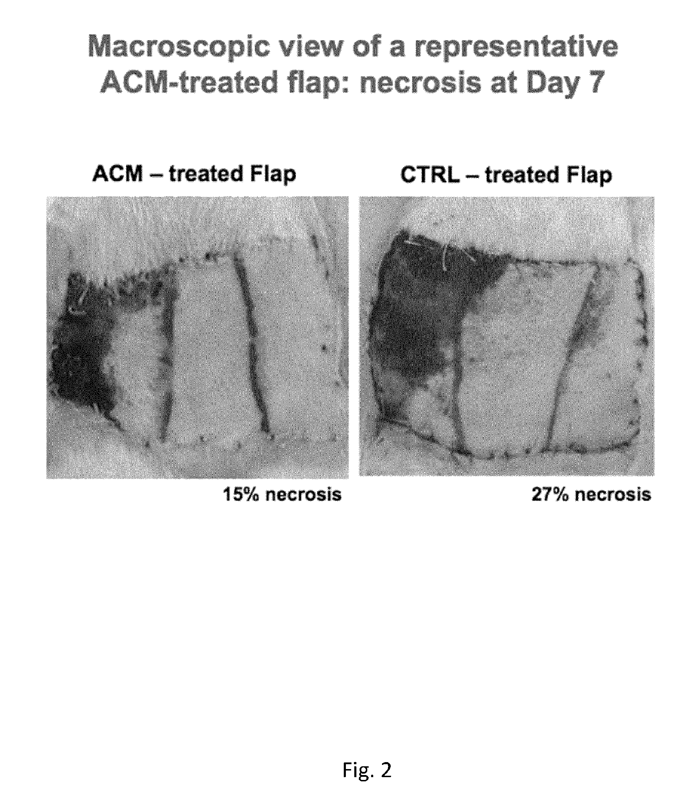

Fig. 2 A macroscopic view of flaps in the conditioned medium containing gelatin membrane treated and in the control (untreated) group of animals at day 7 after ligation of the epigastric bundle.

### EXAMPLES

### Example 1

A 20 mL solution of gelatin (Sigma Aldrich, G8150)/water at a ratio of
a. 5/95 w/w (5% gelatin)
b. 10/90 w/w (10% gelatin)
c. 15/85 w/w (15% gelatin)
d. 25/75 w/w (25% gelatin)
is prepared by pouring under stirring the gelatin powder into water at 50°C. The solution is filtered at 0,22 µm to remove the suspended particles and for sterilization. The filtered solution is then poured at 50°C into a suitable mold (3-4 mm thickness) and cooled at 25°C to induce gelification.

After cooling, half of the samples for each preparation (from 5 to 25 % as indicated above as a. to d.) is also aged at 29°C for 24 hours. The remaining half is kept at room temperature (20-23°C).

### Example 2

A 20 mL solution of 15/85 w/w gelatin/water is prepared as in example 1 at 50°C and poured into a suitable mold. The solution is then dried in a ventilated oven at 60°C for 24 hours.

The heat dried sample is then reconstituted to hydrogel (15% w/w gelatin) by addition of 17 mL water and used 1 hour (a.) or 24 hours (b. aged) after the addition of water.

### Example 3

A 15% w/w gelatin hydrogel is prepared as in example 1 into a suitable mold. The hydrogel is then lyophilized at -50°C under vacuum (0,3 mbar) for 48 hours.

The lyophilized sample is then reconstituted to hydrogel (15% w/w gelatin) by addition of 17 mL water and used 1 hour (a.) or 24 hours (b. aged) after the addition of water.

### Example 4

The physical resistance (elastic modulus E) of samples from the examples 1-3 is assessed on a 0,5 inch diameter gel by means of an Instron 4502 at a testing speed of 1,3 mm/min at 23°C and 33°C. The results are reported in Table I.

**Table I: Physical resistance of gel samples obtained according to Examples 1-3**

| Preparation (example) | Gelatin (% w/w) | E (KPa) at 23°C | E (KPa) at 33°C |
|---|---|---|---|
| 1 a. | 5 | 6,9 | 2,7 |
| 1 a. aged | 5 | 7,0 | 2,7 |
| 1 b. | 10 | 34,9 | 2,9 |
| 1 b. aged | 10 | 34,7 | 2,9 |
| 1 c. | 15 | 79,6 | 23,2 |
| 1 c. aged | 15 | 79,9 | 22,9 |
| 1 d. | 25 | 188,4 | 75,7 |
| 1 d. aged | 25 | 188,6 | 75,0 |
| 2 a. | 15 | 78,8 | 22,9 |
| 2 b. aged | 15 | 79,4 | 23,4 |
| 3 a. | 15 | 80,0 | 23,5 |
| 3 b. aged | 15 | 79,4 | 23,1 |

The results show that aging and the two processes (heat-drying, Example 2 or lyophilization, Example 3) do not affect the mechanical properties of the reconstituted gel, compared at the same gelatin concentration in the hydrogel. As expected, the physical resistance of the hydrogels increases by increasing the gelatin concentration in it.

### Example 5

Melting temperature (gel-sol transition) of samples from the examples 1-3 is determined by means of Differential Scanning Calorimetry (DSC) at 1°C/min scan rate using a Mettler DSC30 calorimeter equipped with a StarE v.6 software. The results are reported in Table II.

**Table II: Melting temperature of gel samples obtained according to Examples 1-3**

| Preparation (example) | Gelatin (% w/w) | Melting Temperature (°C) |
|---|---|---|
| 1 a. | 5 | 34,5 |
| 1 a. aged | 5 | 37,1 |
| 1 b. | 10 | 34,5 |
| 1 b. aged | 10 | 37,8 |
| 1 c. | 15 | 36,2 |
| 1 c. aged | 15 | 37,9 |
| 1 d. | 25 | 37,5 |
| 1 d. aged | 25 | 38,6 |
| 2 a. | 15 | 36,0 |
| 2 b. aged | 15 | 37,8 |
| 3 a. | 15 | 36,2 |
| 3 b. aged | 15 | 38,0 |

The results show that the aging slightly increase (by 1-2°C) the melting temperature of the reconstituted gel. The slight difference is statistically meaningful, since DSC is a very precise and discriminating method especially at the experimental scan rate. The results will drive the inventors to the selection of the most suitable hydrogel and its preparation process aimed to the use in the targeted organ.

The two processes (heat-drying, Example 2 or lyophilization, Example 3) do not affect the melting temperatures of the reconstituted gel, compared at the same gelatin concentration in the hydrogel.

### Example 6

Samples having 35 mm diameter and 4 mm thickness from the examples 1-3 are poured in water at 37°C to determine the dissolution rate. The results are reported in table III.

**Table III: Dissolution rate of samples obtained according to Examples 1-3**

| Preparation (example) | Gelatin (% w/w) | Dissolution rate |
|---|---|---|
| 1 a. | 5 | <20 sec |
| 1 a. aged | 5 | <1 min |
| 1 b. | 10 | <20 sec |
| 1 b. aged | 10 | <1 min |
| 1 c. | 15 | <20 sec |
| 1 c. aged | 15 | <1 min |
| 1 d. | 25 | <1 min |
| 1 d. aged | 25 | <2 min |
| 2 a. | 15 | <10 min |
| 2 b. aged | 15 | <2 min |
| 3 a. | 15 | <10 min |
| 3 b. aged | 15 | <2 min |

Compared to the reference samples, aging allows a faster dissolution, therefore a faster release of the biologically active substances, or a comparable dissolution rate. The two processes (heat-drying, Example 2 or lyophilization, Example 3) slightly decrease the dissolution, compared at the same gelatin concentration in the hydrogel. A modulation on the release rate (immediate or almost immediate) is therefore possible by selecting the process variables.

### Example 7

A 15% w/w gelatin hydrogel is prepared according to the example 3 (gel formation + lyophilization + reconstitution). Platelet Rich Plasma (PRP) is used instead of water to form the gel by cooling from a solution at 42°C. PRP is prepared from pooling buffy coats preparations which are centrifuged at low speed in order to separate the platelet poor plasma (PPP) from the platelet pellet. After the centrifugation the PPP fraction is removed while the platelet pellet is diluted with a defined volume of PPP in order to have a PRP preparation with a platelet concentration between 1x10⁶ and 10x10⁶ platelets/µL. Water is used for reconstitution from the lyophilized form. The physical resistance, melting temperature and dissolution rate in water at 37°C are determined 1 hour or 24 hours (aged) after the addition of water according to the methods reported in examples 4, 5 and 6, respectively. Results are reported in table IV.

**Table IV: Physical resistance, melting temperature and dissolution rate of PRP/gelatin gel**

| Preparation (example) | Gelatin in PRP (% w/w) | E (KPa) at 23°C | E (KPa) at 33°C | Melting temperature | Dissolution rate |
|---|---|---|---|---|---|
| 3 a. | 15 | 79,6 | 23,5 | 36,0°C | <10 min |
| 3 b. aged | 15 | 79,4 | 23,1 | 36,1°C | <2 min |

The addition of PRP in the system does not modify the characteristics of the reference hydrogel, such as physical resistance, melting temperature and dissolution rate.

### Example 8

A 15% w/w gelatin hydrogel is prepared according to the example 3. A human Amniotic Fluid Stem Cells (AFSCs) conditioned medium (ACM) was used instead of water. Culture medium was: αMEM medium (Gibco, Milan, Italy) containing 15% ES-FBS, 1% glutamine and 1% penicillin/streptomycin (Gibco), supplemented with 18% Chang B and 2% Chang C (Irvine Scientific, Santa Ana, CA, USA), Conditioned medium (ACM) was collected and stored in 5 milliliter aliquots equivalent to the medium conditioned by 4 X 10⁶ AFSC. This concentration (1 ml medium conditioned by 800.000 AFSC during a 16 hour culture) was used.

The physical resistance, melting temperature and dissolution rate in water at 37°C are determined 1 hour or 24 hours (aged) after the addition of water according to the methods reported in examples 4, 5 and 6, respectively. Results are reported in table V.

**Table V: Physical resistance, melting temperature and dissolution rate of conditioned medium (ACM) /gelatin gel**

| Preparation (example) | Gelatin in ACM (% w/w) | E (KPa) at 23°C | E (KPa) at 33°C | Melting temperature | Dissolution rate |
|---|---|---|---|---|---|
| 3 a. | 15 | 79,2 | 23,1 | 36,2°C | <10 min |
| 3 b. aged | 15 | 79,0 | 23,0 | 36,2°C | <2 min |

The addition of a cell conditioned culture medium in the system does not modify the characteristics of the reference hydrogel, such as physical resistance, melting temperature and dissolution rate.

### Example 9

A 15% w/w gelatin hydrogel is prepared according to the example 3. A 2% w/w acyclovir (Yung Zip Chemical Co. Ltd) aqueous solution is used instead of water. The physical resistance, melting temperature and dissolution rate in water at 37°C are determined 1 hour or 24 hours (aged) after the addition of water according to the methods reported in examples 4, 5 and 6, respectively. Results are reported in table VI.

**Table VI: Physical resistance, melting temperature and dissolution rate of acyclovir/ gelatin gel**

| Preparation (example) | Gelatin in acyclovir (% w/w) | E (KPa) at 23°C | E (KPa) at 33°C | Melting temperature | Dissolution rate |
|---|---|---|---|---|---|
| 3 a. | 15 | 78,8 | 22,9 | 36,0°C | <10 min |
| 3 b. aged | 15 | 79,3 | 23,4 | 36,2°C | <2 min |

The addition of a drug (acyclovir) in the system does not modify the characteristics of the reference hydrogel, such as physical resistance, melting temperature and dissolution rate.

### Example 10

A 15% w/w gelatin hydrogel is prepared according to the example 3. A 5% w/w paclitaxel (Yung Zip Chemical Co. Ltd) acetone/water 60/40 v/v solution is used instead of PRP. The physical resistance, melting temperature and dissolution rate in water at 37°C are determined 1 hour or 24 hours (aged) after the addition of water according to the methods reported in examples 4, 5 and 6, respectively. Results are reported in table VII.

**Table VII: Physical resistance, melting temperature and dissolution rate of paclitaxel/gelatin gel**

| | | | | | |
|---|---|---|---|---|---|
| Preparation (example) | Gelatin in paclitaxel (% w/w) | E (KPa) at 23°C | E (KPa) at 33°C | Melting temperature | Dissolution rate |
| 3 a. | 15 | 79,8 | 23,6 | 35,9°C | <10 min |
| 3 b. aged | 15 | 80,2 | 23,4 | 36,3°C | <2 min |

The addition of a drug (paclitaxel) in the system does not modify the characteristics of the reference hydrogel, such as physical resistance, melting temperature and dissolution rate.

### Example 11 - Microcapsules

Gelatin microcapsules, having an average size of 300 µm, (15% w/w gelatin) are prepared by adding gelatin powder, previously finely grounded to an average size of 50 µm in a mortar, in a suitable amount of water (from 2 to 10 g of water per gram of gelatin) at 20°C under stirring. At 20°C, the gelatin does not dissolve but only swell in contact with water.

15% w/w gelatin microcapsules are also prepared as above using PRP or a human Amniotic Fluid Stem Cells (AFSCs) conditioned medium (ACM) or a 2% w/w acyclovir aqueous solution or a 5% w/w paclitaxel acetone/water solution instead of water. The melting temperature and the dissolution rate in water at 37°C are determined according to the methods reported in examples 5 and 6, respectively.

### Results are reported in table VIII.

**Table VIII: Melting temperature and dissolution rate of gelatin microcapsules.**

| Preparation | Collagen (% w/w) | Melting temperature | Dissolution rate |
|---|---|---|---|
| Gelatin/water microcapsules | 15 | 36,0°C | <20 sec |
| Gelatin/PRP microcapsules | 15 | 36,0°C | <20 sec |
| Gelatin/conditioned medium micro-cps | 15 | 36,1°C | <20 sec |
| Gelatin/2% w/w acyclovir microcapsule | 15 | 36,2°C | <20 sec |
| Gelatin/5% w/w paclitaxel | 15 | 35,8°C | <20 sec |

The dissolution rate of the microcapsules is immediate and comparable to that of similar single unit hydrogels.

### Example 12 - In vivo effect

A 15% w/w gelatin hydrogel is prepared according to the example 1. PRP is used instead of water to form the gel by cooling from a solution at 42°C. The solution is poured into a 20 x 20 mm casting container for gelling to have a final thickness of 2-3 mm.

A 15% gelatin hydrogel reference (Gelatin & Protein Co., Ltd, GP type), having a gel-sol transition temperature higher than 42°C, is prepared in the same way using PRP. Both the hydrogels, here in form commonly defined as "membranes", are implanted in mice with a stimulated necrosis model, as detailed in Example 14.

The mice are sacrificed 6 hours, 24 hours and 7 days after implantation. The recovery from necrosis and the presence of inflammation in the surrounding area are evaluated by means of histology. The results are reported in the Table IX.

**Table IX: Necrosis and inflammation grades in PRP/gelatin hydrogels.**

| Time | Reference membrane | | Hydrogel membrane according to example 1 | |
|---|---|---|---|---|
| | % necrosis | Inflammation | % necrosis | Inflammation |
| 6 hours | 100 | Poor | 90 | No |
| 24 hours | 95 | Poor | 75 | No |
| 7 days | 70 | No | 50 | No |

The hydrogels according to invention showed a better in vivo performance (lower necrosis and no inflammation) compared to those with hydrogels having a higher melting temperature.

### Example 13 In vivo skin regeneration

The same hydrogel preparation and reference membranes of Example 12 are evaluated in hairless mice with severe burning on the skin (experimental reference: J.M. Stevenson, R.L. Gamelli, R. Shankar, Mouse Model of Burn Wounding and Sepsis, Methods in Molecular Medicine, 1, Volume 78, Wound Healing, I, Pages 95-105).

The hydrogel membranes (30 x 30 mm) according to the invention are poured onto the skin every 24 hours for 7 consecutive days.

While the reference membrane remain intact, the hydrogel membrane according to the invention partially melts in 24 hours. The remaining part of the partially melted membrane which remains onto the burned skin after 24 hours is then removed by pouring warm water at 38-40°C; the lesion is left to dry and then a new membrane is poured onto it.

The recovery from the burning is evaluated by determining the grade (%) of remaining burning. The burning extension is evaluated optically, by making a picture of the burned wound, evaluating the area still burned and normalizing this value to the area at time zero. The recovery is the complementary value to the burning (higher recovery = lower burning).

The results are reported in table X.

**Table X: Burning grades in PRP/gelatin hydrogels after 7 days.**

| | Hydrogel according to example 1 | Reference |
|---|---|---|
| % Burning | 45 | 75 |

The hydrogels according to invention showed a better recovery (lower burning grade) compared to the hydrogels having a higher melting temperature.

### Example 14

Mirabella T et al. (25) recently reported the pro-angiogenic properties of the conditioned medium obtained from human amniotic liquid derived stem cells (ACM). The capability of the lyophilized gelatin membrane loaded with this conditioned medium to promote new blood vessel formation, to restore the normal perfusion levels in an ischemic damaged area and to reduce the necrotic area was tested in a rat model of ischemic full-thick skin flap elevated on the epigastric region (Ref. Michlits W, Mittermayr R, Schafer R, Redl H, Aharinejad S. Fibrin-embedded administration of VEGF plasmid enhances skin flap survival. Wound Repair Regen. 2007;15(3):360-367).

In the experimental animal group the lyophilized gelatin membrane is reconstituted before the use with the cell culture conditioned medium while in the control animal group the lyophilized gelatin membrane is reconstituted before the use with distilled water.

The authors determined the vascular perfusion rate, the vessel distribution and the survival of flaps treated with membranes containing conditioned medium (ACM) (ACM-treated flaps) and demonstrated the ACM-mediated recruitment of endothelial-like progenitors.

The Ischemic Sectors of ACM treated flaps showed at Day 7 a perfusion level 50% higher than the pre-operation baseline, while the not treated control group never recovered the initial perfusion.

The consequent necrosis developed in the Ischemic Sector was delayed and significantly lower in the ACM group. The histology of the ACM-treated flaps revealed a thin dead stratum corneum, a normal arrangement of epidermal and dermal structures and a high density of vessels in subcutaneous tissues. The authors also found that ACM recruited endothelial progenitors (CD31+/VEGFR2+ and CD31+/CD34+ cells) into the ischemic subcutaneous tissues.

## Claims

1. A gelatin gel comprising between 5 and 25 % w/w of gelatin able to melt at a temperature between 34°C and 39°C for medical use.

2. The gelatin gel according to claim 1 wherein the gelatin is dissolved in a solvent comprised in the group of: water, blood and/or plasma derivatives, conditioned cell culture medium, cell suspension containing cell culture medium, therapeutically and/or biologically active agent solution.

3. The gelatin gel according to claim 2 wherein the blood and/or plasma derivatives is selected in the group of: Platelet-Rich-Plasma (PRP), Platelet-Lysate (PL), and/or Platelet Poor Plasma (PPP), and/or Cryoprecipitate (CRYO).

4. The gelatin gel according to claim 2 wherein the conditioned cell culture medium is conditioned by human stem cells.

5. The gelatin gel according to claim 4 wherein the human stem cells are human amniotic liquid derived stem cells.

6. The gelatin gel according to any of previous claims for use as a tissue regenerating agent.

7. The gelatin gel according to claim 6 wherein the tissue is skin, derma and/or connective tissue.

8. The gelatin gel according to claims 6 or 7 wherein the use as a tissue regenerating agent is further to a necrosis or a burning event.

9. The gelatin gel according to claims 6 or 7 for wound or bed sores healing.

10. The gelatin gel according to claims 6 to 9 for topical administration.

11. The gelatin gel according to claim 1 for use as an anti-adhesion agent and/or as a barrier.

12. The gelatin gel according to claim 2 wherein the therapeutically and/or biologically active agent solution is a growth factor.

13. The gelatin gel according to claim 2 wherein the therapeutically and/or biologically active agent solution is selected from the group of: an anti-tumor agent, an anti-bacterial and/or an anti-microbial agent, an anti-viral agent, an anti-inflammatory agent.

14. The gelatin gel according to any of previous claims being in a flat shape or in a tridimensional shape.

15. The gelatin gel according to any of previous claims being dehydrated or lyophilized.
